# EUROPEAN PATENT SPECIFICATION

(11) **EP 1 888 138 B1**
(45) Date of publication and mention of the grant of the patent: **04.09.2013**
(21) Application number: 06770045.0
(22) Date of filing: 05.05.2006
(51) Int. Cl.: A61L 27/54, A61L 31/16, A61L 33/10, A61F 2/06

(54) **IMPREGNATED POLYMER COMPOSITIONS AND DEVICES USING THEM**
IMPRÄGNIERTE POLYMERZUSAMMENSETZUNGEN UND GERÄTE DAMIT
COMPOSITIONS DE POLYMERE IMPREGNE ET DISPOSITIFS COMPRENANT CES COMPOSITIONS

(30) Priority: 10.05.2005 US 127058
(43) Date of publication of application: 20.02.2008
(73) Proprietor: Abiomed, Inc., Danvers, Massachusetts 01923 (US)
(72) Inventor: STEWART, Robert, B., Ipswich, Massachusetts 01938 (US); VAUGHAN, Stephen, Medford, Massachusetts 02155 (US); BOLT, William, Beverly, Massachusetts 01915 (US)
(74) Representative: Gill, Siân Victoria
(86) International application number: PCT/US2006/017501
(87) International publication number: WO 2006/121908

(56) References cited:
- EP-A- 1 462 127
- EP-A1- 0 920 843
- EP-A2- 0 953 320
- EP-A2- 1 479 401
- WO-A-91/08790
- WO-A-2004/014449
- WO-A2-99/38547
- WO-A2-2005/018683
- US-A- 5 342 621
- US-A1- 2005 079 132

## Description

### FIELD OF THE TECHNOLOGY

Certain examples disclosed herein relate to medical devices formed from compositions with impregnated agents, and, more particularly, certain examples relate to medical devices that comprise a composition that includes a polyurethane and an impregnated pharmacological agent.

### BACKGROUND

Thrombosis remains an ongoing issue with long term blood contacting devices. Such devices have been coated to attempt to address the thrombosis issue. In some circumstances, though, alternative or additional techniques may be needed to provide a desired level of treatment.
Medical devices formed of a biomedical polymer having an antithrombogenic agent incorporated therein or coated therewith are described in 455, 342, 621.

### SUMMARY

Certain features, aspects and examples disclosed herein are directed to medical devices formed compositions that include an impregnated agent. Such compositions may also include the same or a different agent coated on a surface of the composition or on the surfaces of a device comprising the composition. Examples of the compositions disclosed herein have numerous uses including, for example, tubing, connectors, stents, catheters, membranes, implants, and artificial organs such as ventricular assist devices, artificial hearts, etc., and other devices that may be used in chemical analysis or in medical procedures or medical therapies. Additional uses of compositions with impregnated agents will be readily selected by the person of ordinary skill in the art, given the benefit of this disclosure.

In accordance with a first aspect, a medical device formed from a composition that includes a polymer and a pharmacological agent impregnated in the polymer is provided. In some examples, the agent may also be coated on one or more surfaces of the composition, e.g., a fluid contacting surface, in addition to being impregnated in the composition. The exact type of polymer and agent in the composition may vary depending on the intended use of the composition and illustrative agents and polymers are described in more detail below. In certain examples, the composition may be effective to provide sustained release of agent for at least a desirable period. In some examples, the agent is an athrombogenic agent to deter, prevent or reduce thrombus formation.

In accordance with a second aspect, a composition that includes a polymer having formula (I) shown below and a pharmacological agent impregnated in the polymer is disclosed. 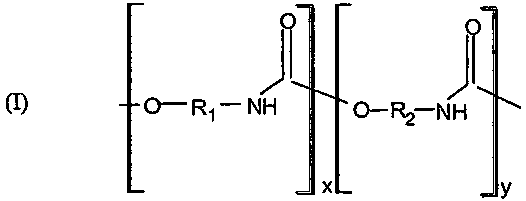 In formula (I), each of R₁, and R₂ may be independently selected from one or more of a saturated hydrocarbon (e.g., a hydrocarbon having 1-6 carbon atoms), an unsaturated hydrocarbon (e.g., a hydrocarbon having 2-6 carbon atoms), a cyclic hydrocarbon, an unsubstituted and substituted phenyl, phenoxy, an amide, a toluene isocyanate, a toluene diisocyanate, and a polyisocyanate. Additional illustrative groups for R₁ and R₂ of formula (I) are listed below. In certain examples of formula (I), x and/or y may independently be between about 10 and 20,000. In some examples, the pharmacological agent may be coated on one or more surfaces of the polymer in addition to being impregnated in the polymer. The exact type of agent in the composition that includes a polymer having formula (I) can vary depending on the intended use of the composition and illustrative agents, e.g., athrombogenic agents, are described in more detail below.

In accordance with an additional aspect, a ventricular assist device constructed and arranged to provide sustained release of pharmacological agent is disclosed. In some examples, the ventricular assist device comprises a polymer and a pharmacological agent impregnated in the polymer. In certain examples, the device may be configured to release an effective amount of a pharmacological agent, e.g., an effective amount of an athrombogenic agent.

These and other features, aspects, examples and uses of compositions that include a polymer and an agent impregnated in the polymer are described in more detail below.

### BRIEF DESCRIPTION OF THE FIGURES

Certain examples are described below with reference to the accompanying figures in which:

FIG. 1 is a schematic of a catheter, in accordance with certain examples; and

FIGS. 2A and 2B are schematics of a ventricular assist device, in accordance with certain examples.

It will be recognized by the person of ordinary skill in the art, given the benefit of this disclosure, that the examples shown in the figures are not necessarily drawn to scale. Certain features or components may have been enlarged, reduced or distorted to facilitate a better understanding of the illustrative aspects and examples disclosed herein. In addition, the use of shading, patterns and the like in the figures is not intended to imply or mean any particular material or orientation unless otherwise clear from the context.

### DETAILED DESCRIPTION

Examples of the medical devices formed from compositions disclosed herein may be used in many medical and applications where it may be desirable to provide sustained or controlled release of agent, and, in particular, examples of the compositions disclosed herein are well-suited to provide sustained or controlled release of agent into fluid, such as blood, lymph, cerebrospinal fluid, bile, urine and the like. The specific agent impregnated in the polymer of the compositions will typically depend on the intended use of the composition. It will be within the ability of the person of ordinary skill in the art, given the benefit of this disclosure, to select suitable agents for impregnating in a polymer of the composition.

In accordance with certain examples, a composition comprising a polymer and a pharmacological agent impregnated in the polymer is provided. In certain examples, the composition may be effective to provide sustained or controlled release of pharmacological agent for at least a desirable period, e.g., 1, 2, 3, 4, 5, 6-12 months or more. In contrast, commonly used coatings on devices may only release agent for less than one day, which can greatly reduce coating effectiveness and generally deters use of coated devices for long term therapies and long term insertion of such devices in mammals, such as humans. Devices that includes the compositions disclosed herein may provide sustained release of pharmacological agents into fluid, such as blood, lymph, cerebrospinal fluid and the like, which allows for use of such devices for long term medical treatment.

As used herein, the term "impregnated" refers to agent being disposed or distributed internally in a polymer, e.g., disposed or distributed in the internal lattice network of the polymer. Unlike a coating, when an agent is impregnated in a polymer there exists little or no observable interface. In some examples, substantially all agent may be impregnated within the polymer of the composition with little or no agent being disposed on an external surface of the polymer of the composition. In other examples, however, agent may be coated or disposed on an external surface of the polymer of the composition in addition to agent being impregnated in the polymer. In certain examples, agent may be impregnated into a polymer of the composition prior to use of the composition to produce a device, e.g., tubing, stents, ventricular assist devices, artificial hearts and the like. In other examples, the device may be first formed using the polymer and the agent may then be impregnated into the device subsequent to, or during, formation of the device. Additional methods of making devices using the illustrative compositions disclosed herein will be readily selected by the person of ordinary skill in the art, given the benefit of this disclosure.

In certain examples, the polymer of the composition may be selected from suitable polymers commonly used in medical devices, chemical analysis and separations, forensic analysis and the like. In certain examples, a biocompatible polymer may be used such that little or no unwanted side effects, e.g., immunogenic reactions, are caused from using the composition in a mammal, such as a human. In some examples, more than one polymer may be used in the composition, and at least one of the polymers may include an impregnated agent. In other examples, a single polymer having identical monomers (e.g., a homopolymer) may be used, whereas in other examples a single polymer having two or more different monomers (e.g., a copolymer) may be used. In certain examples, the polymer of the compositions may be linear, branched, cross-linked or take other forms commonly found in polymers. In some examples, the polymers may be an addition polymer or a condensation polymer. The polymer may contain charged groups and the overall charge on the polymer may be positive, negative or neutral. In certain examples, the polymer may be hydrophobic, oleophobic, hydrophilic or amphipathic.

Without wishing to be bound by any particular scientific theory or this example, suitable polymers for use in the composition disclosed here may include an internal lattice structure that is suitable for receiving and trapping agent. As fluid contacts the composition with impregnated agent, the impregnated agent may elute or diffuse out of the polymer lattice and into the fluid to provide a desired physiological, pharmacological or therapeutic effect. The exact type of polymer may vary depending on the intended use of the compositions. In certain examples, the polymer may be selected from one or more of a polyether, a polyurethane, a polyesterurethane, a polyetherurethane, a polyetherurethaneurea, a polyester, a polycarbonate, a low density polyethylene, a medium density polyethylene, a high density polyethylene, a polyethylene terephthalate, a polyvinyl chloride, a polypropylene, a polystyrene, a polyamide, a polyacrylamide, a polyacrylate or combinations thereof. Such illustrative polymers are commercially available from numerous suppliers including, for example, Noveon (Cleveland, OH), Sigma-Aldrich (St., Louis, MO) and the like. Illustrative polyamides may be synthesized by reaction between a diacid and a diamine, e.g., adipic acid and hexamethyldiamine. Illustrative polyesters may be synthesized by reaction between a diacid and a dialcohol, e.g., dimethyl terephthalate and ethylene glycol. Exemplary polycarbonates may be prepared by reaction between a carbonate and an alcohol or phenol, e.g., diphenyl carbonate and Bisphenol A. Polyethylenes, polypropylenes, polyvinyl chlorides, polystyrenes, etc. may be prepared by radical polymerization of alkenes, by cationic polymerization of alkenes, or anionic polymerization of alkenes. Additional methods for preparing polymers suitable for use in the instant disclosure will be readily selected by the person of ordinary skill in the art, given the benefit of this disclosure.

In some examples, the polymer may include at least two monomers selected from ethylene, a haloethylene (e.g., fluoroethylene, chloroethylene, bromoethylene, iodoethylene), propylene, styrene, tetrafluoroethylene, acrylonitrile, methyl methacrylate, vinyl acetate, a vinyl alcohol, a vinyl halide, a substituted and an unsubstituted phenyl, phenoxy, butadiene, and a styrene. Additional polymers and additional monomers for use in producing polymers will be readily selected by the person of ordinary skill in the art, given the benefit of this disclosure. Particularly suitable polymers for use in the compositions include, but are not limited to polymers having the following trade names: Biomer®, Texin®, Tecoflex®, Tecothane®, Carbothane®, Tecophilic®, Estane®, EstaGrip®, Estaloc®, Tecoplast®, Europrene® Kraton®, Vector®, Solprene®, Translute® and Stereon® polymers, which are available commercially from various suppliers. Other suitable commercially available polymers will be readily selected by the person of ordinary skill in the art, given the benefit of this disclosure.

In accordance with certain examples, a polymer having a weight average molecular weight of about 50,000 to about 1,000,000, more particularly from about 100,000 to about 200,000, e.g., about 120,000 to about 180,000 may be used in the compositions disclosed here. In other examples, a polymer having a number average molecular weight of about 30,000 to about 250,000, more particularly about 50,000 to about 100,000, e.g., about 60,000 to about 85,000, may be used in the compositions disclosed here.

In certain examples, the composition may also include additives, e.g., dyes, colorants, fillers, elastomers, indicators and the like, impregnated in the polymer. For example, the polymer may include an impregnated indicator to provide rapid determination that sufficient amounts of agent are still available for release from the composition. The indicator may be detected using suitable chemical, biological and/or biochemical methods. It will be within the ability of the person of ordinary skill in the art, given the benefit of this disclosure, to select suitable additives for including in the compositions disclosed here.

In some examples, the polymer may be synthesized in the presence of a volatile liquid, such as fluorotrichloromethane, dichloromethane, etc., such that a polymer foam is formed. In certain examples, the polymer may be produced in the presence of a volatile liquid and the agent to be impregnated so that as the polymer foam is formed the agent may become impregnated in the polymer foam. The person of ordinary skill in the art, given the benefit of this disclosure, will be able to select additional suitable methods of making polymer foams.

In accordance with certain examples, a composition that includes a polymer having formula (I) shown below and an agent impregnated in the polymer is disclosed. 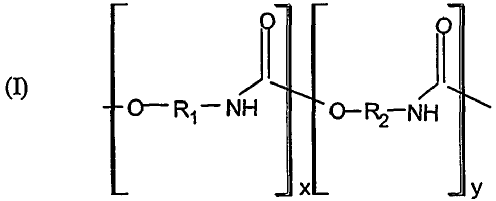 In formula (I), each of R₁ and R₂ may be independently selected from one or more of a saturated hydrocarbon (e.g., a hydrocarbon having 1-6 carbon atoms, more particularly 1-3 carbon atoms), an unsaturated hydrocarbon (e.g., a hydrocarbon having 2-6 carbon atoms, more particularly 2-4 carbon atoms), a cyclic hydrocarbon, an unsubstituted phenyl, a substituted phenyl, a phenoxy, an amide, a toluene isocyanate, a toluene diisocyanate, and a polyisocyanate. In certain examples of formula (I), x and/or y may be between about 10 and about 10,000, more particularly between about 10 and about 5,000, e.g., between about 10 to about 2,000 or any number within these illustrative ranges.

In some examples, a polymer having formula (I) is produced from a diol (or polyol) and a toluene diisocyanate, polyisocyanate, or the like, and the chemical make-up of R₂ depends, at least in part, on the selected diol (or polyol) and the chemical make-up of R₁ depends, at least in part, on the selected toluene diisocyanate, or polyisocyanate. Initiators, chain extenders, catalysts and the like may also be used to produce the polymer. The diols or polyols may include primary, secondary or tertiary hydroxyl groups. Suitable diols and polyols are commercially available from numerous suppliers, such as Sigma-Aldrich (St. Louis, MO), the Olin Corporation (Cheshire, CT), and Bayer AG (Leverkusen, Germany). Illustrative polyols include, but are not limited to Arcol® polyols (Bayer AG), Poly-L® polyols (Olin Corporation) and the like. Illustrative isocyanates include, but are not limited to, di-functional or polyfunctional isocyanates, such as those commercially available from Bayer AG (Leverkusen, Germany), BASF Corporation (Parsippany, N.J.), The Dow Chemical Company (Midland, MI), and Huntsman Chemical (Utah). Exemplary polyisocyanates include, but are not limited to diphenylmethane-4,4'-diisocyanate (MDI), toluene-2,4-diisocyanate (TDI), toluene-2,6-diisocyanate (TDI), methylene bis (4-cyclohexylisocyanate (H₁₂ MDI), 3-isocyanatomethyl-3,5,5-trimethyl-cyclohexyl isocyanate (IPDI), 1,6-hexane diisocyanate (HDI), naphthalene-1,5-diisocyanate (NDI), 1,3-and 1,4-phenylenediisocyanate, triphenylmethane-4,4',4"-triisocyanate, polyphenylpolymethylene-polyisocyanate (PMDI), m-xylene diisocyanate (XDI), 1,4-cyclohexyl diisocyanate (CHDI), isophorone diisocyanate, isomers and mixtures or combinations thereof. In certain examples, R₁ includes a terminal oxygen moiety that is bonded to an additional monomer in the polymer chain, and R₂ includes a terminal carbonyl moiety that is bonded to an additional monomer in the polymer chain. Exemplary methods for producing polymers are described, for example, in U.S. Patent No. 6,734,273, the entire disclosure of which is hereby incorporated herein by reference for all purposes.

In certain examples and for biocompatibility, either MDI or H₁₂ MDI may be reacted with polytetramethyleneoxideglycol (PTMEG) in the presence of a chain extender, such as, for example, 1,4-butanediol, to form an unbranched polymer structure. This reaction may result in polymers with excellent biocompatibility properties. Using these reagents, a polymer of formula (I) may result where each of R₁ and/or R₂ may independently have a chemical structure as shown in formula (II) below. 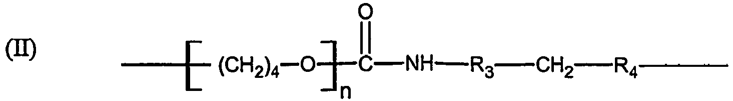 In formula (II), R₃ and R₄ may each be aromatic (when MDI is used) or aliphatic (when H₁₂ MDI is used). In certain examples, n is about 1-500. In other examples, n may be about 1-5 for one of the monomers, whereas n may be 20 to 500 for the other monomer in formula (I). The polymer may also have a distribution of monomer sizes (n in Formula II) for R₁ and/or R₂ in formula (I).

In accordance with certain examples, another class of a thermoplastic elastomer that may be used is a block co-polymer that includes a polystyrene moiety separated by a conjugated diene moiety. The conjugated diene may be fully or partially hydrogenated, or may include mixtures thereof. Generally, these block-copolymers may contain about 10-35 weight %, e.g., 10-25 weight %, of styrene and about 75 to about 35 weight % of the conjugated diene, based on the block-copolymer. Specific block-copolymers of the styrene/conjugated diene/styrene-type that may be used are SBS (styrene-butadienestyrene), SIS (styrene-isoprene-styrene), SIBS (styrene-isobutylene-styrene), SEBS (styrene-ethylene/butylene-styrene) and SEPS (styrene-ethylene/propylene-styrene, and SEEPS (styrene-ethylene/ethylene/propylene-styrene).

In certain examples, a polymer may be, or may include, a block co-polymer such as, for example, the generic polymeric structure suitable for block polystyrene co-polymers shown below in formula (III), wherein R₅ may be a diene, e.g., a conjugated diene (or dienes), that is incorporated into the polymer backbone. Illustrative dienes include, but are not limited to, butadiene, polybutadiene, isoprene, polyisoprene, chloroprene, and polychloroprene. Additional dienes will be readily selected by the person of ordinary skill in the art, given the benefit of this disclosure. In certain examples, a and/or b and/or c in formula (III) may independently be between about 10 and about 1,000, more particularly between about 10 and about 500, e.g., between about 10 and 100. 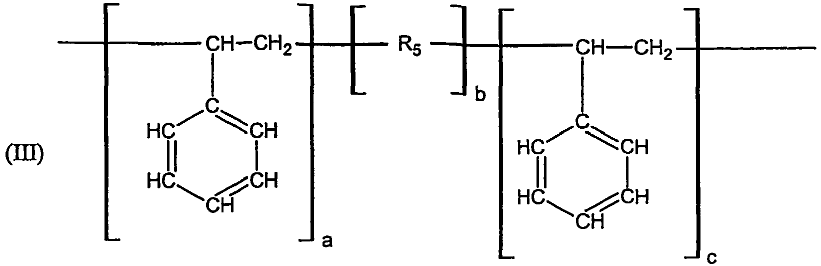 In certain examples, the impregnated composition may include a block polystyrene co-polymer, an athrombogenic agent, e.g., a heparin, and a complexing agent such as, for example, benzalkonium chloride. Other athrombogenic agents and complexing agents suitable for use with a polymer that includes a block co-polymer will be readily selected by the person of ordinary skill in the art, given the benefit of this disclosure.

In accordance with certain examples, one or more pretreatment steps may be performed on the polymer prior to impregnation of the agent in the polymer. Such pretreatment steps may render the polymers more compatible or susceptible with the agent and may remove residual impurities. Removed impurities may include, but are not limited to, catalysts, partially reacted, low molecular weight polymer components or unreacted polymer components. In addition, the percentage of hard and soft segments of the finished polymer may be varied by processing the raw polymers. Many polymer processing methods may be employed including extraction with suitable organic solvents, reprecipitation, ultrafiltration, or other polymer purification methods. In addition, the polymer may be purified by heating to the melt temperature, then filtered or otherwise processed to remove unwanted components, for instance by first extruding the polymer, after which the polymer is dissolved and solvent cast. Suitable methods for treating the polymer to remove impurities and/or render the polymer more susceptible to impregnation will be readily selected by the person of ordinary skill in the art, given the benefit of this disclosure. Exemplary citations that describe purification methods include, but are not limited to, Lelah et al. Trans. ASAIO, 1981, 504-510; Marchant et al. J. Biomed. Mater. Res., 1986, 799-815; and Nurdin et al. J. Biomater. Sci. Polym. Ed., 1995, 49-60.

In accordance with certain examples, the agent impregnated in the polymer of the composition may vary depending on the intended use of the composition. The impregnated agent is referred to in some instances herein as the "active agent." For example, where the composition is used in implants, medical devices, ventricular assist devices, artificial hearts and the like that include surfaces that contact blood, an athrombogenic agent, e.g., an anticoagulant, a thrombolytic agent, an antiplatelet agent, or combinations thereof, may be impregnated in the polymer of the composition. As used herein, "athrombogenic agent" refers to any agent that can act to prevent, deter or reduce thrombus formation on one or more surfaces or in the blood. Additional agents, other than athrombogenic agents, may also be impregnated in the polymer. For example, where the composition is used in surgical screws, fasteners and the like, growth factors, for example, may be impregnated in the polymer of the composition.

The loading rate of agent is 1 % to 5 % by weight based on the total solid weight of the composition,

In certain examples, the agent may be an anticoagulant agent. In some examples, the anticoagulant agent may be a heparin. Illustrative examples of a heparin include, but are not limited to, human heparin (natural or recombinant forms), truncated forms of human heparin, animal heparin (e.g., bovine and porcine heparin), heparin sulfate, a low molecular weight heparin (1 to 10 kDa, e.g., enoxaprin, Lovenox®, dalteprin, Fragmin®, fondaparineux (brand name Arixtra®)), a heparin analogue, a synthetic heparin, a heparanoid (e.g., Orgaran®), and/or combinations thereof. Human heparin is a mixture of glycosaminoglycans with an average molecular weight of about 15,000 Daltons. The heparin molecule acts as a catalyst in the neutralization reaction between anti-thrombin (AT-III) and thrombin (Th), thereby preventing fibrin formation. When AT-III binds to heparin, the AT-III:Th reaction kinetics are increased 1000-fold. In addition, the removal of Th also inhibits the Th-induced activation of other coagulation enzymes. Further, heparin also increases the binding of AT-III to several coagulation enzymes. As a result of its catalytic action and multi-inhibitory properties, minute amounts of heparin can significantly reduce thrombus formation. Without wishing to be bound by any particular scientific theory or this example, porcine heparin may lower risk of heparin induced thrombocytopenia in humans when compared to bovine heparin.

In other examples, the anticoagulant agent may be coumarin, 4-hydroxycoumarin, bishydroxycoumarin, warfarin, phenprocoumon, indan-1,3-dioneacenocoumarol, anisindione, or hirudin (an anticoagulant peptide from *Hirudo medicinalis,* in either its natural form or in any recombinant form).

In certain examples, the agent may be a thrombolytic agent. Illustrative thrombolytic agents include, but are not limited to, plasminogen, alpha2-antiplasmin, streptokinase, tissue plasminogen activator, urokinase, and aminocaproic acid. In some examples, the agent may be an antiplatelet agent. Illustrative antiplatelet agents include, but are not limited to, aspirin, dipyridamole and ticlopidine. Additional thrombolytic and antiplatelet agents will be readily selected by the person of ordinary skill in the art, given the benefit of this disclosure.

In other examples, an agent that includes an amino acid, a nucleoside, a nucleoside phosphate, a growth factor, an antibody, a vitamin, an antibiotic, an antiviral, an angiogenic agent, a chemotherapeutic, or other suitable therapeutic or pharmacological agent may be used to treat a particular disease or disorder. The person of ordinary skill in the art, given the benefit of this disclosure, will be able to select suitable agents to accomplish a desired physiological or pharmacological effect.

In accordance with certain examples, the agent may be complexed with a complexing agent prior to, during or subsequent to impregnation of the agent into the polymer to form the composition. Without wishing to be bound by any particular scientific theory or this example, a complexing agent may be used to increase solubility of the active agent in the selected solvent system used to produce the composition. For example, many active agents are water soluble and have limited solubility in some of the solvents, e.g., tetrahydrofuran, that may be used to produce the composition. In some examples, the complexing agent may be added to provide a 1:1 ratio of complexing agent:active agent, whereas in other examples excess complexing agent (e.g., as high as a 100:1 ratio of complexing agent:active agent) may be used to ensure that substantially all of the active agent is complexed.

The loading rate of the complex formed from the complexing agent and the active agent is 1 % to 5 % by weight based on the total solid weight of the composition.

In accordance with certain examples, the exact nature of the selected complexing agent depends, for example, on the selected solvent system, the active agent to which the complexing agent is intended to form a complex with, and the solubility of the active agent in the solvent system. In certain examples, the complexing agent may be amphipathic having one end that may complex with active agent and a second end that may act to increase solubility of the complex in the solvent system. Illustrative complexing agents include, but are not limited to, benzalkonium salts (e.g., benzalkonium chloride), ammonium salts, tridodecylammonium chloride (TDMAC), cetylpyrimidine chloride, sterylammonium chlorides (e.g., benzylsterylammonium chlorides), combinations thereof and the like. To prepare the active agent:complexing agent complex, numerous procedures may be used. For example, a solution of active agent, typically an aqueous solution, may be mixed vigorously with a solution of complexing agent, typically in an organic solvent. The active agent:complexing agent complex preferably remains in the organic phase of the mixture. When the organic phase is separated, and the solvent is allowed to evaporate, the active agent:complexing agent complex may be recovered. In other examples, the active agent:complexing agent complex may precipitate, e.g., spontaneously, into the aqueous phase of the mixture. After the active agent:complexing agent complex is recovered, it may be dissolved in a suitable solvent, e.g., THF, prior to mixing with the polymer to form the composition.

In accordance with certain examples, a suitable solvent system may be used to prepare the composition. As used herein, "solvent system" refers to the solvent or solvents used to dissolve or solvate the polymer, complexing agent and/or the active agent. The exact solvent system may vary and is typically selected based on the properties, e.g., polarity and/or solubility, of the selected polymer and the selected agent. In some examples, the solvent system includes one or more of tetrahydrofuran (THF), dimethyl sulfoxide (DMSO), dimethylacetamide (DMAC), dioxane, ethanol, methanol, propanol, isopropanol, ethyl ether, toluene and/or other mineral spirits, alkanes, cycloalkanes, and freons, including, for example, dichloroethane and trichloroethane. Preferably the solvent system is "inert" so that no unwanted side reactions occur between the polymer and the solvent, the agent and the solvent, or the complexing agent and the solvent. In certain examples, pure THF, dioxane, dimethylacetomide (DMAC) or methylene chloride (MC) may be used as the solvent system. In other examples, mixtures of solvents may be used to provide different drying times for polymer compositions. For example, MC may be added to THF to decrease the drying time (compared to the drying time using THF neat), or dioxane may be added to THF to lengthen the cure time of polymer compositions (compared to the cure time using THF neat). Shorter cure times may be desirable to reduce the overall time to produce, for example, whole cast parts, while longer drying times may be desirable to obtain a more even cure, for example, through an entire film layer. Additional solvent systems will be readily selected by the person of ordinary skill in the art, given the benefit of this disclosure.

In accordance with certain examples, a composition comprising a polymer and an agent impregnated in the polymer may be produced by dissolving a suitable amount of polymer in a suitable solvent system. For example, a sufficient amount of polymer may be added to provide about 1-30% by weight polymer, e.g., about 1-20% or 1-10% by weight polymer, based on the weight of the solvent system. After the polymer is dissolved or suspended in the solvent system, a suitable amount of active agent (or complexed active agent) may be added to the dissolved or suspended polymer. For example, a sufficient amount of active agent (or complexed active agent) may be added to provide 1+05 weight % active agent, based on the total solid weight. Mixing of the active agent (or complexed agent) and the composition is allowed for a suitable time for the active agent to become impregnated in the polymer. Mixing may be accomplished with conventional techniques such as magnetic stir bars and stir plates, vortex mixing, agitation, stirring, and the like. The composition may then be used along with suitable forming processes to produce desired devices. For example, solvent casting (dip casting), injection molding, extrusion or the like may be used to produce a desired device. Illustrative devices are discussed in more detail below.

In accordance with certain examples, a pharmacological agent may be homogeneously incorporated into an outermost layer of a blood contacting composition. This may be accomplished using a two step process. For example, a drug or drug complex may be disposed on the composition, or polymer part made from the composition, e.g., by dipping or otherwise exposing the composition to a solution containing a drug or drug complex. The solvent used to dissolve the drug or drug complex may be volatile and evaporate rapidly. Evaporation of the solvent will leave a coating of the drug or drug complex. A second solvent may then be used that dissolves both the drug or drug complex and the polymer. Use of the second solvent allows for the drug or drug complex to be homogeneously incorporated into the outermost layer of the composition, or polymer part made from the composition. The second solvent step may be accomplished by dipping the coated polymer part into the second solvent, though this process may reduce the ultimate concentration of drug in the outer polymer layer. To prevent reduction in drug concentration, the second solvent may be heated above its boiling point, and the coated polymer part may be exposed to the solvent vapors. This step is similar to solvent polishing a polymer part. The solvent vapor may dissolve both the polymer and the drug or drug complex to provide a surface layer with a maximum concentration of drug or drug complex. When the two step process is completed properly, a clear and colorless part may be manufactured. By impregnating the pharmacological agent into a surface layer, an extended therapeutic effect should be possible.

In accordance with certain examples, tubing comprising a composition that includes a polymer and an agent impregnated in the polymer is provided. In some examples, the tubing may be formed by mixing the polymer and the agent in a suitable solvent system and dipping a rod into the composition to form a film on the rod. The film may be air dried, or dried in an oven, and the rod may be re-dipped in the composition to increase the thickness of the tubing. Illustrative tubing thicknesses include, but are not limited to, about 0.1 mm to about 5 mm outer diameter. Additional tubing thicknesses will be readily selected by the person of ordinary skill in the art, given the benefit of this disclosure. The tubing may also be formed using injection molding, extrusion processes and the like. The polymer and agent used in the composition to form the tubing may be any of the illustrative polymers and agents described herein or other suitable polymers and agents. The selected agent typically depends, at least in part, on the disease or condition to be treated. For example, where the tubing is used in cardiac catheterization processes, the tubing may include an athrombogenic agent or other suitable agents commonly used in the treatment of cardiac disorders. In some examples, the tubing includes one or more heparins, such as the illustrative heparins described herein. It will be within the ability of the person of ordinary skill in the art, given the benefit of this disclosure, to select suitable agents for use in tubing and devices including tubing.

In accordance with certain examples, an intra-aortic balloon that includes a polymer and an agent impregnated in the polymer is provided. An illustrative intra-aortic balloon is described in commonly owned U.S. Patent No. 5,090,957.
Without wishing to be bound by any particular scientific theory or this example, intra-aortic balloons (IABs) may be used to support the circulation during periods of reduced heart performance. The IAB may be placed in the blood stream in the aorta or pulmonary artery. Existing IABs can be prone to thrombus development, in particular when the IAB is used in closed spaces or in the weaning mode where the IAB is periodically folded onto itself. By producing an IAB that includes a polymer having an impregnated agent, e.g., an athrombogenic agent, the device is less prone to thrombus development. Again without wishing to be bound by any particular scientific theory or this example, the athrombogenic agent may inhibit thrombus formation on the surface of the balloon by inhibiting the coagulation reaction on the surfaces of the IAB. In some examples, only the blood contacting surfaces of the IAB are made using a polymer having an impregnated agent, whereas in other examples substantially all of the IAB is made from a polymer having an impregnated agent. The person of ordinary skill in the art, given the benefit of this disclosure, will be able to design suitable IABs that include the compositions disclosed herein.

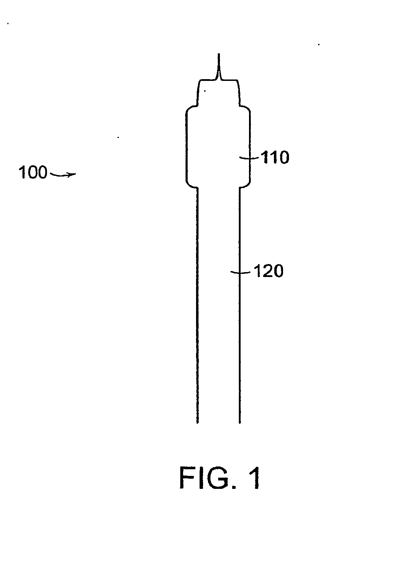

Referring now to FIG. 1, an exemplary IAB is shown. IAB 100 includes a balloon or bladder 110 mounted at the tip of an inflation tube 120. In some examples, the balloon has dimension of about 0.5 to about 2 cm in diameter and about 15 cm to about 40 cm in length and is initially uninflated. In order to insert the balloon, it may be folded or otherwise compacted so that its maximum diameter is approximately that of the inflation tube, or about three to six millimeters, for example. In the conventional Seldinger technique, an IAB may be inserted via a minor artery by first using a guide wire and dilator to establish a path to the desired location in the aorta, and extending a sheath and dilator along the guide wire to its end. The dilator may be then removed, leaving the sheath in place. Finally, the folded or wrapped balloon may be inserted by pushing its inflation tube through the sheath, thus positioning the balloon at the desired spot prior to inflation. By constructing the IAB using a polymer having an athrombogenic agent impregnated in the polymer, thrombus formation on IAB surfaces can be prevented and/or reduced.

In accordance with certain examples, a ventricular assist device (VAD) that comprises a composition that includes a polymer and an agent impregnated in the polymer is provided. In some examples, the ventricular assist device is constructed and arranged to provide sustained or long term release of athrombogenic agent over a desired period, e.g., 1 month to 1 year or more. An exemplary ventricular assist device is described in commonly owned U.S. Patent No. 4,782,817, and an exemplary artificial heart is described in commonly owned U.S. Patent No. 4,888,011. Without wishing to be bound by any particular scientific theory or this example, thrombosis remains an ongoing issue with long term blood contacting devices and especially for VADs. Long term release of an athrombogenic agent at the blood/device interface may be achieved using the compositions disclosed herein, which can lead to a reduction in complications in the use of VADs. In addition, *in situ* therapy is also possible. In contrast to coatings, which can be depleted in less than 1 day and can have issues with fatigue caused by long term flexing, the compositions disclosed herein may provide for sustained release of impregnated agent to provide a long term therapeutic effect.

In accordance with certain examples, temporary VADs are currently being used for cardiac support times ranging from days to months. Two blood pump designs are currently being used clinically. One design involves the use of a highly textured surface, which actively promotes rapid protein and cellular deposition and eventual tissue ingrowth. A textured surface is used on a VAD, the Heartmate®, which has been approved by the FDA for long term implantation for destination therapy for patients in end stage congestive heart failure, A second blood pump design, used in several VADs approved for temporary use for both bridge-to-recovery and bridge-to-transplant indications, involves the use of a smooth surface, which relies on optimal flow patterns in the pump "washing" the surface clean. However, minute flow stagnation caused by surface imperfections, or temporary low flow conditions can significantly degrade the washing capabilities of the flow field, resulting in potential sites for thrombus formation. The primary complications with smooth surface VADs are secondary to thrombus formation on the artificial surfaces of the blood contacting materials incorporated in the device. Oral or intravenous anti-coagulation therapy is currently used to control the rate of thrombus formation; however, thromboembolic (TE) events remain problematic for VADs in long term use (e.g., > 1 month). The person of ordinary skill in the art, given the benefit of this disclosure, will be able to design suitable VADs that include the compositions disclosed herein, and an illustrative VAD is described below.

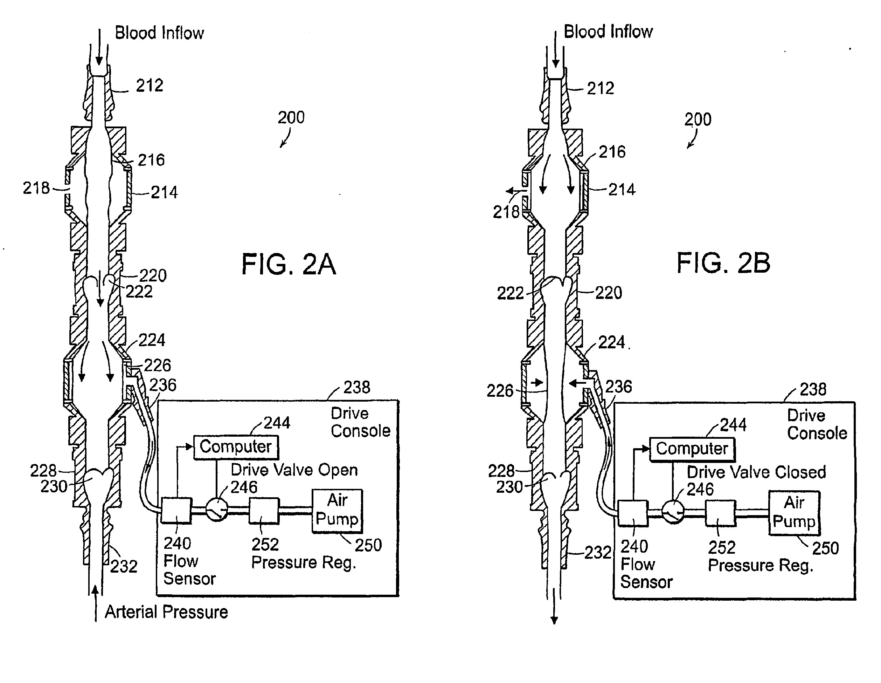

In accordance with certain examples, a composition comprising a polymer and a pharmacological agent may be used to fabricate a cardiac assist device. An illustrative assist device is shown in FIGS. 2A and 2B. Referring now to FIG. 2A, assist device 200 may be configured for diastole, that is that portion of the cycle when the blood inflow is filling the bladder of the active portion of the pump prior to the systolic ejection of blood from the pump. The illustrative device shown in FIG. 2A consists of two major subsystems: a drive console 238 and a single-use disposable blood pump. The blood pump, like the natural heart, may be comprised of two chambers 214 and 224 each including a flexible bladder, 216 and 226 respectively, and two valves 222 and 230. Some or all of the components of assist device 200 may be made from a polymer, e.g., a polyurethane, that includes an impregnated agent, e.g., an athrombogenic agent. The upper chamber 214 may be configured as a filling chamber or atrium, while the lower chamber 224 may be configured as a pumping chamber or ventricle. The pumping bladder 226 may be isolated from the bladder 216 of the inflow chamber 214 and systemic pressures by one polymeric trileaflet valve 222 at its entrance and a second polymeric trileaflet valve 230 at its exit. One or both of trileaflet valves 222 and 230 may be made from a composition that includes a polymer, e.g., a polyurethane, and an impregnated agent, e.g., an athrombogenic agent. The inflow chamber 214 may be connected via tubing and cannula to the natural atrium of the patient's heart. Filling of the pump may be continuous and passive as a result of atrial pressure and gravity. This result may be accomplished by lowering the device below the patient's atrial level, typically by less than about 20 cm. The outflow chamber 224 may be emptied by air pulses delivered from the drive console 238, and may be filled from the inflow chamber blood volume as the air is vented through the console. As shown in FIG. 2A, the input port 212 is in fluid communication with the patient, e.g., an artery or vein of the patient, and may convey blood from the patient into the inflow bladder 216 situated within a generally rigid walled first chamber 214. This chamber may be vented through opening 218 to the atmosphere allowing, in this portion of the cycle, air to flow into the chamber while the bladder 216 is in a generally collapsed condition. It should be noted that the bladder 216 may not be completely collapsed but may remain open to allow blood from the blood inflow port 212 to pass through it and through the open trileaflet valve 222 contained in trileaflet valve housing 220. The blood inflow may be mainly provided by gravity. In this diastole mode, the outlet valve 230 may remain closed since its bias is such that the force of the gravity and the atrial pressure is insufficient to open it. The outlet valve 230 may be in fluid communication with an output port 232 which in turn is connected to the arterial system of the patient.

In certain examples, the drive console 238 may include an air pump 250 which may generate pressurized air coupled through a pressure regulator 252 to an electromagnetically controlled drive valve 246. The air pump will typically produce about 20-60 psi pressure, and the pressure regulator 252 may be arranged to produce a pressure of approximately 250 mm Hg when operated as a left ventricle and about 200 mm Hg when operated as a right ventricle. The output of the controlled valve 246 may be connected through flow sensor 240 to the pneumatic tube 236. In the position shown for diastole, however, the valve 246 does not couple the air pressure to the pneumatic tube 236, but rather blocks off the pressure from the air pump 250 and opens the pneumatic tube 236 to the atmosphere, thereby venting through tube 236 the exterior portion of chamber 224. Flow sensor 240 may be any suitable volumetric flow sensor, for example, a constricted orifice with a differential pressure measuring device. The output from the flow sensor 240 may be connected to a computer 244 which contains software for controlling the operation of the entire support system. This computer provides a control signal back to electromagnetic valve 246 controlling when that valve may be in the open position (as shown) or in the closed position as illustrated in FIG. 2B. During the diastolic portion of the cycle the air may be driven from the exterior portion of the chamber 224 as blood fills the bladder 226 and this flow of air provides a signal from the sensor 240 to the computer 244 indicative of the flow of blood through the blood inflow into the bladder 226.

Referring now to FIG. 2B, the same elements are shown in the configuration for pump systole as those shown in FIG. 2A. In this configuration the valve 246 may be closed, coupling pressurized air from the pump 250 through the regulator 252 and the tubing 236 to the outer portion of the chamber 224 to compress the outflow bladder 226 and thereby forcing the blood which accumulated during the diastolic portion of the cycle out through the valve 230 to the arterial system of the patient. As a result of the pressurization of the internal volume of the bladder 226, the valve 222 may close so that blood can be ejected only through the outflow to the patient and not return into the first chamber bladder 216. However, even while this pressurization is proceeding, blood inflow may still be passing into the inflow bladder 16 and accumulating there in preparation for the next cycle.

In accordance with certain examples, all of the blood contacting surfaces of the mechanical pump shown in FIGS. 2A and 2B, that is the input and output ports, may be formed from a composition including a polymer and an agent impregnated in the polymer. In certain examples, the polymer is a polyurethane and the agent is an athrombogenic agent. In some examples, the polymer is a polyurethane polymer, such as, for example Biomer®, Texin®, Tecoflex®, Tecothane®, Carbothane®, Tecophilic®, Estane®, EstaGrip®, Estaloc®, Tecoplast®, Europrene® Kraton®, Vector®, Solprene®, Translute®, Stereon® polymers or other commercially available polymers. In certain examples, the agent is a heparin, such as bovine heparin or porcine heparin. The heparin may be complexed with a complexing agent, e.g., BAC, or may be uncomplexed. Additional suitable polymers and agents for use in compositions present in cardiac assist devices will be readily selected by the person of ordinary skill in the art, given the benefit of this disclosure. In some examples, the volumes of bladders 216 and 226 are each about 100 cubic centimeters producing an output stroke volume of about 80 cubic centimeters. Typical values for the patient connections are 1/2 inch inner diameter tubing.

In accordance with certain examples, control of the device shown in FIGS. 2A and 2B is typically accomplished using a computer and software, as described more completely, for example, in U.S. Patent No. 4,782,817, the entire disclosure of which is incorporated herein by reference for all purposes.

In accordance with certain examples, the compositions disclosed herein may also be used in the various components of an artificial heart. Exemplary artificial heart components are described, for example, in U.S. Patent No. 4,888,011, U.S. Patent No. 5,084,064, U.S. Patent No. 6,319,231, U.S. Patent No. 6,324,431, U.S. Patent No. 6,442,434, U.S. Patent No. 6,445,956, U.S. Patent No. 6,496,733, U.S. Patent No. 6,527,698, U.S. Patent No. 6,533,724, and U.S. Patent No. 6,540,658. .

In certain examples, heparin may be complexed with a complexing agent, e.g., benzalkonium chloride (BAC). When mixed properly, clear and colorless devices, e.g., tubing, VADs and the like, can be manufactured, that incorporate the heparin:BAC complex homogeneously throughout the device. It should be understood, however, that there is no requirement that the devices be clear and colorless, but overall clarity can allow for visual or optical inspection of devices (e.g., for bubbles in the composition), and can avoid occlusions in the composition, which may result in either a rough surface, or a potential sub-surface nucleation site for calcification. Also, by forming the entire blood contacting part(s) from a polymer composition with impregnated agent, polymer flex life is no longer an issue, as long as the bulk material properties of the composition remain within acceptable limits.

In accordance with certain examples, a VAD may be produced using any of the illustrative polymers described herein. In some examples, the VAD is produced from a polyurethane polymer, and in particular, a high molecular weight polyether-based polyurethane polymer such as Biomer® and Cardiothane-51. Using solvent casting methods, intricate, yet highly durable and flexible cardiac assist devices can be constructed. In certain examples, heparin complexed with a complexing agent may be used to produce the composition used in the VADs. The complexed heparin may be loaded with polymer to obtain a solution with about 3-20% by weight solids, e.g., about 10% solids, based on the weight of the solvent. Both heparin:TDMAC (STS Biopolymers) and heparin:BAC (Sigma-Aldrich) complexes are commercially available. The heparin may be complexed with other complexing agents. The resulting complex may be mixed with a polymer, in either powder or solution form, in a suitable solvent system to provide the final composition.

In accordance with the present application, the loading of the agent has little effect on the tensile properties of the polymer. For example, using loading rates of 1-5%, or more preferably from 2-3%, the tensile strength should not significantly differ, when compared to base polymer without agent and when determined using standard tensile measuring methods. An acceptable test method is a non-destructive stress:strain measurement, such as applying a linear elongation force to thin strips of the agent impregnated polymer resulting in up to 100% elongation (using, for example, ASTM Method 412). Appropriate dimensions for the thin strips may be about 1 cm wide by about 5 cm long by about 0.05 cm thick. Without wishing to be bound by any particular scientific theory or this example, in repetitive flexing during VAD use the polymer may undergo less than about 10% strain. If the Young's modulus is determined over this range, with the tensile test mentioned above, the change in the Young's modulus should be less than 10%, and preferably less than 3% either above or below the Young's modulus obtained when testing thin strips made with the base polymer (i.e., without any impregnated agent).

In accordance with certain examples, to test the suitability of compositions for use in VADs and other medical devices, the release (elution) rate of different polymer compositions impregnated with drugs may be varied. For short term VAD or device use, release of the drug or drug complex over days to a few weeks may be desirable. For longer term implanted VADs, release rates of months up to several years may be desirable. Either the drug or drug complex may be released over these time periods. For optimal effect, the drug should be released such that it is in a biologically active form. As an example, for heparin complexed with other molecules, such as a benzalkonium:heparin complex, it may be preferable for the complexing agent to remain in the polymer, while the heparin may be released independently of the complex. Heparin released in this manner may have an optimal effect for prevention of thrombus formation on VADs or other medical devices.

In accordance with certain examples, elution rates of agents may be measured into saline or other biological fluids, and as an example, may be determined by circulating the chosen fluid through a flow loop incorporating tubes, or other structures made with the polymer compositions impregnated with agent. Measurement of the elution rates may be obtained at different time points by analyzing aliquots taken from the flow loop to determine the concentration of drug per unit of volume. By knowing the total fluid volume in the flow loop and the exposed area of the agent impregnated polymer, the elution rate per unit area per unit time may be obtained. There may be a minimum drug elution rate associated with certain biological effects, e.g., the minimum heparin elution rate needed to maintain patency in a vascular shunt model, as described in Lin et al. "Minimum heparin release for non-thrombogenicity." Trans ASAIO 1987;33:602-605, is 0.5 U/(hour cm²).

In certain examples, the optimal heparin elution rate for use VADs and other medical devices manufactured with examples of agent impregnated polymer disclosed herein may be substantially different than the rate given by Lin et al., and may depend upon effects such as, for example, flow fields through the devices, and other related factors, including the administration of anti-coagulants or other drugs to prevent thrombus. For an example of the latter case, if a VAD made with polymer compositions impregnated with agent, e.g., heparin, is used in conjunction with Anti-thrombin III therapy, an increase in the local concentration of the agent by elution at the VAD polymer surface may potentiate the ability of Anti-thrombin III to inhibit thrombus formation. Similarly, other systemically administered drugs may also interact with the eluted drugs for various desirable local effects. It will be within the ability of the person of ordinary skill in the art, given the benefit of this disclosure, to select suitable systemically administered drugs which may be potentiated by the local elution of an agent, or an agent-complex, from a polymer surface with an objective, for example, of changing the local environment of the device.

In accordance with certain examples, the rate of agent elution from the polymer may be dependent on many factors. Generally, the elution rate may be directly dependent on the amount of the agent or agent-complex impregnated in the polymer. In addition, the molecular size of the agent or agent-complex may influence the elution rate, as diffusion of a given agent through the polymer may vary based on its molecular size. As an example, a polymer impregnated with lower molecular weight heparins and heparin analogues may elute the chosen anti-coagulants much faster than polymers impregnated with unfractionated heparin. Furthermore, base polymer characteristics including, for example, hydrophilicity, crystallinity and cross-link density may affect the elution rate. For instance, water soluble drugs may have a higher elution rate in hydrophilic polymers. In addition, more crystalline or higher cross-linked polymer may be associated with lower agent elution rates. It will be within the ability of the person of ordinary skill in the art, given the benefit of this disclosure, to select suitable agents and agent complexes and corresponding polymers for use to obtain desired drug or drug complex elution rates.

In accordance with certain examples, tri-leaflet valves may be manufactured in a normal manner. Details for manufacturing such tri-leaflet valves, may be found, for example, in U.S. Patent No 4,888,009. Without wishing to be bound by any particular scientific theory, when tri-leaflet valves are produced using one or more impregnated polymer compositions, e.g., a polymer having a heparin:BAC complex, the performance of the tri-leaflet valve should not differ substantially from the performance of a tri-leaflet valve including the base polymer with no impregnated agent.

In accordance with certain examples, preliminary reliability testing may be performed on various components of a VAD, such as a polyurethane tri-leaflet valve. For example, it may be desirable to complete a limited durability study to verify that no short to medium term effects are associated with impregnating the polymer with the heparin. Reliability testing may be completed using a commercially available heart valve tester (Hi-Cycle System, Model No. HCS4991 from ViVitro Systems, Victoria, British Columbia, Canada). Using the instructions provided with the tester, up to 6 artificial heart valves may be cycled for long periods of time under physiological temperature, pressure and flow conditions. Accelerated testing may be completed using rates up to 10 times real time using this system. The use of this system for testing heart valves is described, for example, in Mackay et al. "In vitro function and durability assessment of a novel polyurethane heart valve prosthesis." Artif. Organs, 20 (1996) 1017-25.

In accordance with certain examples, a VAD that includes a composition comprising a polymer and an agent impregnated in the polymer and agent coated on at least one VAD surface is disclosed. In some examples, the agent is coated onto at least one blood contacting surface of the VAD in addition to being impregnated in the polymer composition of the VAD. Without wishing to be bound by any particular scientific theory or this example, while the agent coating may not provide sustained release of agent, the coating may increase initial serum levels of eluted agent immediately subsequent to VAD insertion. Illustrative coatings and methods for applying such coatings are described in U.S. Patent No. 5,525,348. The polymer compositions disclosed herein may also be used in an artificial heart that includes an agent coated on at least one surface of the artificial heart.

In accordance with certain examples, medical devices comprising a composition that includes a polymer and an impregnated agent are provided. The exact configuration of the medical devices will typically depend on the intended use of the device, and illustrative medical devices include surgical screws, surgical fasteners, intramedullary nails, dental posts, vertebral fusion devices, venous stents, urethral stents, spinal stents, and blood line connectors. An illustrative device is described in commonly owned U.S. Patent No. 6,445,956. It will be within the ability of the person of ordinary skill in the art, given the benefit of this disclosure, to design suitable medical devices that include the compositions comprising a polymer and an agent impregnated in the polymer.

### Examples

Certain specific examples are described below to illustrate further examples, aspects and features of the technology described herein.

### Example 1

A composition was prepared according to the following method. Porcine heparin complex (heparin:benzalkonium (Sigma-H7280) obtained commercially from Sigma) was dissolved in 100 mL of tetrahydrofuran (THF) (neat) obtained commercially from Sigma in a 250 mL Pyrex® beaker. The mixture was stirred overnight at room temperature using a magnetic stir plate and a magnetic stir bar. 1% by weight Carbothane® PC-3595A (obtained commercially from Thermedics Polymer Products a division of Noveon (Cleveland, OH)), based on the weight of the solvent, was added to the beaker containing the porcine heparin:benzalkonium complex in THF, and the resulting mixture was mixed thoroughly. Additional increments of 1% by weight Carbothane® PC-3595A were added until a level of 10% by weight Carbothane® PC-3595A, based on the weight of the solvent, was achieved.

The resulting composition exhibited the properties shown in Table I below. Solution clarity was determined visually using a four point scale from Poor (highly opaque) to Fair (highly scattering and/or milky in consistency) to Good (minimal scattering and/or a slight turbidity) to Excellent (crystal clear). Cast part clarity was also determined by visual inspection, as set forth above. Heparin activity was determined by the Azure A Dye test as described in Gebauer, B. et al. "Detection of heparin during FVIII isolation using improved azure A method," Acta Phrm 49 (1999) 35-41. Control parts cast from the same polymer solutions (i.e. without heparin:benzalkonium complex) all tested negative for heparin activity using the Azure A dye test.

**Table I**

| Property | Results |
|---|---|
| Solution Clarity | Fair |
| Cast Part Clarity | Good |
| Heparin Activity | Yes |

### Example 2

Another composition was prepared according to the following method. Porcine heparin complex (heparin:benzalkonium (Sigma-H7280) obtained commercially from Sigma) dissolved in 100 mL of dimethylacetamide (DMAC) (neat) obtained commercially from Sigma in a 250 mL Pyrex® beaker. The mixture was stirred overnight at room temperature using a magnetic stir plate and a magnetic stir bar. 1% by weight Biomer® (previously sold by Ethicon (Somerville, NJ)), based on the weight of the solvent, was added to the beaker containing the porcine heparin:benzalkonium complex in DMAC, and the resulting mixture was mixed thoroughly. Additional increments of 1% by weight Biomer® were added until a level of 10% by weight Biomer ®, based on the weight of the solvent, was achieved.

The resulting composition exhibited the properties shown in Table II below. Solution clarity, cast part clarity and heparin activity were determined using the methods described in Example 1 above. Control parts cast from the same polymer solutions (i.e. without heparin:benzalkonium complex) all tested negative for heparin activity using the Azure A dye test.

**Table II**

| Property | Results |
|---|---|
| Solution Clarity | Excellent |
| Cast Part Clarity | Excellent |
| Heparin Activity | Yes |

### Example 3

Another composition was prepared according to the following method. Porcine heparin complex (heparin:benzalkonium (Sigma-H7280) obtained commercially from Sigma) dissolved in 100 mL of tetrahydrofuran (THF) (neat) obtained commercially from Sigma in a 250 mL Pyrex® beaker. The mixture was stirred overnight at room temperature using a magnetic stir plate and a magnetic stir bar. 1% by weight Estane® 5714 (obtained commercially from Noveon (Cleveland, OH)), based on the weight of the solvent, was added to the beaker containing the porcine heparin:benzalkonium complex in THF, and the resulting mixture was mixed thoroughly. Additional increments of 1% by weight Estane® 5714 were added until a level of 10% by weight Estane® 5714, based on the weight of the solvent, was achieved.

The resulting composition exhibited the properties shown in Table III below. Solution clarity, cast part clarity and heparin activity were determined using the methods described in Example 1 above. Control parts cast from the same polymer solutions (i.e. without heparin:benzalkonium complex) all tested negative for heparin activity using the Azure A dye test.

**Table III**

| Property | Results |
|---|---|
| Solution Clarity | Excellent |
| Cast Part Clarity | Excellent |
| Heparin Activity | Yes |

### Example 4

An additional composition was prepared according to the following method. Porcine heparin complex (heparin:benzalkonium (Sigma-H7280) obtained commercially from Sigma) dissolved in 100 mL of tetrahydrofuran (THF) (neat) obtained commercially from Sigma in a 250 mL Pyrex® beaker. The mixture was stirred overnight at room temperature using a magnetic stir plate and a magnetic stir bar. 1% by weight Tecoflex® EG-93A (obtained commercially from Noveon (Cleveland, OH)), based on the weight of the solvent, was added to the beaker containing the porcine heparin:benzalkonium complex in THF, and the resulting mixture was mixed thoroughly. Additional increments of 1% by weight Tecoflex® EG-93A were added until a level of 10% by weight Tecoflex® EG-93A, based on the weight of the solvent, was achieved.

The resulting composition exhibited the properties shown in Table IV below. Solution clarity, cast part clarity and heparin activity were determined using the methods described in Example 1 above. Control parts cast from the same polymer solutions (i.e. without heparin:benzalkonium complex) all tested negative for heparin activity using the Azure A dye test.

**Table IV**

| Property | Results |
|---|---|
| Solution Clarity | Good |
| Cast Part Clarity | Excellent |
| Heparin Activity | Yes |

### Example 5

Another composition was prepared according to the following method. Porcine heparin complex (heparin:benzalkonium (Sigma-H7280) obtained commercially from Sigma) was dissolved in 100 mL of tetrahydrofuran (THF) (neat) obtained commercially from Sigma in a 250 mL Pyrex® beaker. The mixture was stirred overnight at room temperature using a magnetic stir plate and a magnetic stir bar. 1% by weight Texin® 990R (obtained commercially from Bayer Corporation (Plastics Division)), based on the weight of the solvent, was added to the beaker containing the porcine heparin:benzalkonium complex in THF, and the resulting mixture was mixed thoroughly. Additional increments of 1% by weight Texan® 990R were added until a level of 10% by weight Texin® 990R, based on the weight of the solvent, was achieved.

The resulting composition exhibited the properties shown in Table V below. Solution clarity, cast part clarity and heparin activity were determined using the methods described in Example 1 above. Control parts cast from the same polymer solutions (i.e. without heparin:benzalkonium complex) all tested negative for heparin activity using the Azure A dye test.

**Table V**

| Property | Results |
|---|---|
| Solution Clarity | Excellent |
| Cast Part Clarity | Excellent |
| Heparin Activity | Yes |

### Example 6

An additional composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Tecophilic® 93-A (obtained commercially from Noveon (Cleveland, OH)), based on the weight of the solvent, is added to the beaker containing the porcine heparin:benzalkonium complex in THF, and the resulting mixture is mixed thoroughly. Increments of 1% by weight Tecophilic® 93-A are added until a level of 10% by weight Tecophilic® 93-A, based on the weight of the solvent, is achieved.

### Example 7

An additional composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Estaloc® 59600 (obtained commercially from Noveon (Cleveland, OH)), based on the weight of the solvent, is added to the beaker containing the porcine heparin:benzalkonium complex in THF, and the resulting mixture is mixed thoroughly. Additional increments of 1% by weight Estaloc® 59600 are added until a level of 10% by weight Estaloc® 59600, based on the weight of the solvent, is achieved.

### Example 8

Another composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Texin® 950-U (obtained commercially from Bayer Corporation (Plastics Division)), based on the weight of the solvent, is added to the beaker containing the porcine heparin:benzalkonium complex in THF, and the resulting mixture is mixed thoroughly. Increments of 1% by weight Texin® 950-U are added until a level of 10% by weight Texin® 950-U, based on the weight of the solvent, is achieved.

### Example 9

An additional composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Estane® 5778 (obtained commercially from Noveon (Cleveland, OH)), based on the weight of the solvent, is added to the beaker containing the porcine heparin:benzalkonium complex in THF, and the resulting mixture is mixed thoroughly. Increments of 1% by weight Estane® 5778 are added until a level of 10% by weight Estane® 5778, based on the weight of the solvent, is achieved.

### Example 10

Another composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Tecothane® 1085A (obtained commercially from Noveon (Cleveland, OH)), based on the weight of the solvent, is added to the beaker containing the porcine heparin:benzalkonium complex in THF, and the resulting mixture is mixed thoroughly. Increments of 1% by weight Tecothane® 1085A are added until a level of 10% by weight Tecothane® 1085A, based on the weight of the solvent, is achieved.

### Example 11

An additional composition may be prepared according to the following method. Porcine heparin complex (heparin:benzalkonium (Sigma-H7280) obtained commercially from Sigma) dissolved in 100 mL of methylene chloride (MC) (neat) obtained commercially from Sigma in a 250 ml Pyrex® beaker. The mixture is stirred overnight at room temperature using a magnetic stir plate and a magnetic stir bar. 1% by weight Makroblend KU 2-7609 (obtained commercially from Bayer Corporation (Plastics Division)) based on the weight of the solvent, is added to the beaker containing the porcine heparin:benzalkonium complex in MC, and the resulting mixture is mixed thoroughly. Increments of 1% by weight Makroblend KU 2-7609 are added until a level of 10% by weight Makroblend KU 2-7609, based on the weight of the solvent, is achieved.

### Example 12

Another composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Carbothane® PC-3585A (obtained commercially from Noveon (Cleveland, OH)), based on the weight of the solvent, is added to the beaker containing the porcine heparin:benzalkonium complex in THF, and the resulting mixture is mixed thoroughly. Additional increments of 1% by weight Carbothane® PC-3585A are added until a level of 10% by weight Carbothane® PC-3585A, based on the weight of the solvent, is achieved.

### Example 13

An additional composition may be prepared according to the following method. Porcine heparin complex (heparin:benzalkonium (Sigma-H7280) obtained commercially from Sigma) dissolved in 100 mL of methylene choride (MC) (neat) obtained commercially from Sigma in a 250 mL Pyrex® beaker. The mixture is stirred overnight at room temperature using a magnetic stir plate and a magnetic stir bar. 1% by weight Apec^{®} 1745 (obtained commercially from Bayer Corporation (Plastics Division)), based on the weight of the solvent, is added to the beaker containing the porcine heparin:benzalkonium complex in MC, and the resulting mixture is mixed thoroughly. Increments of 1% by weight Apec^{®} 1745 are added until a level of 10% by weight Apec^{®} 1745, based on the weight of the solvent, is achieved.

### Example 14

Another composition may be prepared according to the following method. This composition contains two polymers. Porcine heparin complex (heparin:benzalkonium (Sigma-H7280) obtained commercially from Sigma) dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Tecophilic® 85-A (obtained commercially from Noveon (Cleveland, OH)), based on the weight of the solvent, is added to the beaker containing the porcine heparin:benzalkonium complex in THF, and the resulting mixture is mixed thoroughly. Increments of 1% by weight Tecophilic® 85-A are added until a level of 10% by weight polymers, based on the weight of the solvent, is achieved.

### Example 15

An additional composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Tecothane® 2085A (obtained commercially from Noveon (Cleveland, OH)), based on the weight of the solvent, is added to the beaker containing the porcine heparin:benzalkonium complex in THF, and the resulting mixture is mixed thoroughly. Increments of 1% by weight Tecothane® 2085A are added until a level of 10% by weight Tecothane® 2085A, based on the weight of the solvent, is achieved.

### Example 16

An additional composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Stereon® 840A (obtained commercially from Firestone Polymers (Akron, OH)), based on the weight of the solvent, is added to the beaker containing the porcine heparin:benzalkonium complex in THF, and the resulting mixture is mixed thoroughly. Increments of 1% by weight Stereon® 840A are added until a level of 10% by weight Stereon® 840A, based on the weight of the solvent, is achieved.

### Example 17

An additional composition may be prepared according to the following method. Porcine heparin complex (heparin:benzalkonium (Sigma-H7280) obtained commercially from Sigma) is dissolved in 100 mL of tetrahydrofuran (THF) (neat) obtained commercially from Sigma in a 250 mL Pyrex® beaker. The mixture is stirred overnight at room temperature using a magnetic stir plate and a magnetic stir bar. 1% by weight Translute® (manufactured by Boston Scientific (Natick, MA)), based on the weight of the solvent, is added to the beaker containing the porcine heparin:benzalkonium complex in THF, and the resulting mixture is mixed thoroughly. Increments of 1% by weight Translute® are added until a level of 10% by weight Translute®, based on the weight of the solvent, is achieved.

When introducing elements of the examples disclosed herein, the articles "a," "an," "the" and "said" are intended to mean that there are one or more of the elements. The terms "comprising," "including" and "having" are intended to be open ended and mean that there may be additional elements other than the listed elements. It will be recognized by the person of ordinary skill in the art, given the benefit of this disclosure, that various components of the examples can be interchanged or substituted with various components in other examples. Should the meaning of the terms of any of the patents or publications incorporated herein by reference conflict with the meaning of the terms used in this disclosure, the meaning of the terms in this disclosure are intended to be controlling.

## Claims

1. A medical device or part of a medical device formed from a composition comprising:
a polymer; and
an effective amount of a pharmacological agent impregnated in the
polymer,
in which the impregnated pharmacological agent is disposed or distributed in the internal lattice network of the polymer wherein the composition comprises 1-5% pharmacological agent by weight of the composition.

2. A device or part of a device according to claim 1, in which the pharmacological agent is an athrombogenic agent.

3. A device or part of a device according to claim 1, in which the polymer is a biocompatible polymer.

4. A device or part of a device according to claim 3, in which the polymer is a polyether, a polyurethane, a polyesterurethane, a polyetherurethane, a polyetherurethaneurea, a polyester, a polycarbonate, a low density polyethylene, a medium density polyethylene, a high density polyethylene, a polyethylene terephthalate, a polyvinyl chloride, a polypropylene, a polystyrene, a polyamide, a polyacrylamide, a polyacrylate, or a combination thereof.

5. A device or part of a device according to claim 2, in which the polymer is a polyurethane and the athrombogenic agent is a heparin.

6. A device or part of a device according to claim 5, in which the heparin is human heparin, bovine heparin, porcine heparin, heparin sulfate, a low molecular weight heparin, a heparin analogue, a synthetic heparin, a heparinoid, or a combination thereof.

7. A device or part of a device according to claim 5, further comprising a heparin coated on at least one surface of the polyurethane.

8. A device or part of a device according to claim 1, further comprising a complexing agent.

9. A device or part of a device according to claim 8 wherein the complexing agent is a benzalkonium salt, an ammonium salt, a ttidodecylammonium chloride, a cetylpyrimidine chloride, a sterylammonium chloride, or a combination thereof

10. A device or part of a device according to claim 2, further comprising a complexing agent.

11. A device or part of a device according to claim 10, in which the pharmacological agent is human heparin, bovine heparin, porcine heparin, heparin sulfate, a low molecular weight heparin, a heparin analogue, a synthetic heparin, a heparinoid, or a combination thereof.

12. A device or part of a device according to claim 10 or 11, in which the polymer is a polyetherurethane or a polyesterurethane, the athrombogenic agent is a heparin and the complexing agent is benzalkonium chloride.

13. A device or part of a device according to claim 1, in which the polymer comprises a compound having formula (I) 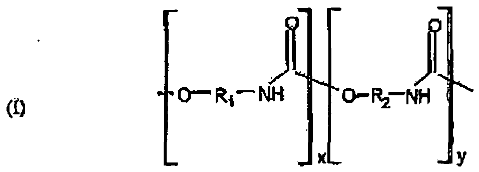 in which each of R₁ and R₂ is independently a saturated hydrocarbon, an unsaturated hydrocarbon, a substituted phenyl, an unsubstituted phenyl, a phenoxy, a toluene isocyanate, a toluene diisocyanate, a polyisocyanate, and wherein x and y are each between 50 to 1,000.

14. A device or part of a device according to claim 13, wherein each of R₁ and R₂ independently comprises a compound having formula (II) 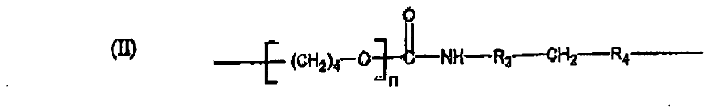 wherein each of R₃ and R₄ is independently an aromatic or an aliphatic cyclic hydrocarbon, and n is between 1 and 500.

15. A device or part of a device according to claim 10, in which the polymer is a block polystyrene co- polymer, the athrombogenic agent is a heparin and the complexing agent is benzalkonium chloride.

16. A device or part of a device according to claim 15, wherein the block polystyrene co-polymer comprises a compound having formula (III) 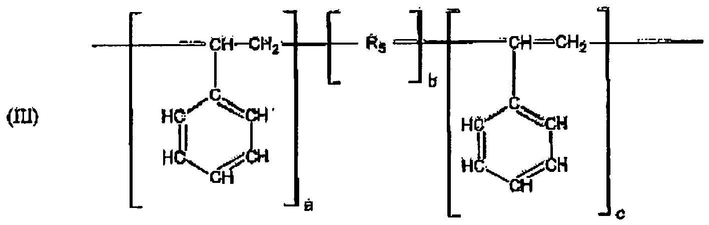 wherein R₅ is a diene and wherein each of a, b and c is independently between 10 and 1,000.

17. A device or part of a device according to claim 13, further comprising an additional pharmacological agent coated on at least one surface of the polymer.

18. A device or part of a device according to claim 13, further comprising a complexing agent.

19. A device or part of a device according to claim 1, comprising 0.05 to 0.5 equivalents of the pharmacological agent per equivalent of the polymer.

20. A device or part of a device according to claim 1, further comprising a surface coating on the composition in which the pharmacological agent has been homogeneously incorporated into the surface coating.

21. A device according to claim 1 constructed and arranged for use as a ventricle of a human heart

22. The device of claim 21, in which the polymer is a polyurethane and the pharmacological agent is a heparin.

23. The device of claim 22, wherein the heparin is complexed with a complexing agent.

24. The device of claim 22, wherein the heparin is human heparin, bovine heparin, porcine heparin, heparin sulfate, a low molecular weight heparin, a heparin analogue, a synthetic heparin, a heparinoid, or a combination thereof.

25. The device of claim 21, further comprising:
a first chamber;
an input port in fluid communication with the first chamber and
configured to allow fluid flow into the first chamber; and
an output port in fluid communication with the first chamber and
configured to allow fluid from the first chamber to an arterial system of a human,
wherein one or both of the input port and the output port comprise a valve.

26. The device of claim 21, further comprising:
a first chamber;
an input port in fluid communication with the first chamber;
a second chamber in fluid communication with the first chamber;
a valve configured to control fluid flow from the first chamber to the second chamber; and
an output port in fluid communication with the second chamber and
configured to provide fluid flow to an arterial system of a human.

27. The device of claim 21, in which all blood contacting surfaces of the device comprise a composition that comprises a polymer and an athrombogenic agent impregnated in the polymer.

28. A ventricular assist device constructed and arranged to provide sustained release of a pharmacological agent, the device comprising a composition comprising:
a polymer; and
an effective amount of a pharmacological agent impregnated in the polymer,
in which the impregnated pharmacological agent is disposed or distributed in the internal lattice network of the polymer, wherein said composition comprises 1-5% pharmacological agent by weight of the composition.

29. The ventricular assist device of claim 28, further comprising a surface coating in which the pharmacological agent has been homogeneously incorporated into the surface coating.

30. The ventricular assist device of claim 28, in which the pharmacological agent is a heparin and the polymer is a polyurethane.

31. The ventricular assist device of claim 28, further comprising:
a first chamber;
an input port in fluid communication with the first chamber and
configured to allow fluid flow into the first chamber; and
an output port in fluid communication with the first chamber and
configured to allow fluid from the first chamber to an arterial system of a human.

32. The ventricular assist device of claim 28, further comprising:
a first chamber;
an input port in fluid communication with the first chamber;
a second chamber in fluid communication with the first chamber;
a valve configured to control fluid flow from the first chamber to the second chamber; and
an output port in fluid communication with the second chamber and
configured to provide fluid flow to an arterial system of a human.

33. A catheter comprising:
a bladder comprising a composition which comprises a polymer impregnated with a first pharmacological agent, in which the impregnated pharmacological agent is disposed or distributed in the internal lattice network of the polymer, wherein the composition comprises 1-5% pharmacological agent by weight of the composition; and
a tube connected to the bladder and operative to inflate the bladder.

34. The catheter of claim 33, in which the first pharmacological agent is a heparin.

35. The catheter of claim 33, in which the bladder comprises a polyurethane.

36. The catheter of claim 35, wherein the bladder further comprises a surface coating comprising a second pharmacological agent homogeneously incorporated in the surface coating, in which the second pharmacological agent may be the same or different than the first pharmacological agent, preferably the first and second pharmacological agents are each a heparin.

37. A device or part of a device according to claim 1 for use in the treatment of thrombosis

38. The device of claim 37, wherein the device is configured as a ventricular assist device.

39. The device of claim 37, wherein the device is configured as a stent.

40. The device of claim 37 wherein the device is configured as a catheter.

## Patentansprüche

1. Medizinische Vorrichtung oder Teil einer medizinischen Vorrichtung, die aus einer Zusammensetzung hergestellt ist, wobei die Zusammensetzung umfasst:
ein Polymer; und
eine wirksame Menge eines pharmakologischen Agens, das in das Polymer imprägniert ist,
wobei das imprägnierte pharmakologische Agens in dem internen Gitternetzwerk des Polymers angeordnet oder verteilt ist, wobei die Zusammensetzung 1-5 % pharmakologisches Agens bezogen auf das Gewicht der Zusammensetzung umfasst.

2. Vorrichtung oder Teil einer Vorrichtung nach Anspruch 1, wobei das pharmakologische Agens ein antithrombogenes Agens ist.

3. Vorrichtung oder Teil einer Vorrichtung nach Anspruch 1, wobei das Polymer ein biokompatibles Polymer ist.

4. Vorrichtung oder Teil einer Vorrichtung nach Anspruch 3, wobei das Polymer ein Polyether, ein Polyurethan, ein Polyesterurethan, ein Polyetherurethan, ein Polyetherurethanharnstoff, ein Polyester, ein Polykarbonat, ein Polyethylen niedriger Dichte, ein Polyethylen mittlerer Dichte, ein Polyethylen hoher Dichte, ein Polyethylen-Terephthalat, ein Polyvinylchlorid, ein Polypropylen, ein Polystyren, ein Polyamid, ein Polyacrylamid, ein Polyacrylat, oder eine Kombination davon ist.

5. Vorrichtung oder Teil einer Vorrichtung nach Anspruch 2, wobei das Polymer ein Polyurethan ist und das antithrombogene Agens ein Heparin ist.

6. Vorrichtung oder Teil einer Vorrichtung nach Anspruch 5, wobei das Heparin menschliches Heparin, Rinderheparin, Schweineheparin, Heparinsulfat, ein Heparin mit niedrigem Molekulargewicht, ein Heparinanalog, ein synthetisches Heparin, ein Heparinoid, oder eine Kombination davon ist.

7. Vorrichtung oder Teil einer Vorrichtung nach Anspruch 5, weiter umfassend ein auf wenigstens einer Oberfläche des Polyurethans beschichtetes Heparin.

8. Vorrichtung oder Teil einer Vorrichtung nach Anspruch 1, weiter umfassend einen Komplexbildner.

9. Vorrichtung oder Teil einer Vorrichtung nach Anspruch 8, wobei der Komplexbildner ein Benzalkoniumsalz, ein Ammoniumsalz, ein Tridodecylammoniumchlorid, ein Cetylpyrimidinchlorid, ein Sterylammoniumchlorid, oder eine Kombination davon ist.

10. Vorrichtung oder Teil einer Vorrichtung nach Anspruch 2, weiter umfassend einen Komplexbildner.

11. Vorrichtung oder Teil einer Vorrichtung nach Anspruch 10, wobei das pharmakologische Agens menschliches Heparin, Rinderheparin, Schweineheparin, Heparinsulfat, ein Heparin mit niedrigem Molekulargewicht, ein Heparinanalog, ein synthetisches Heparin, ein Heparinoid, oder eine Kombination davon ist.

12. Vorrichtung oder Teil einer Vorrichtung nach Anspruch 10 oder 11, wobei das Polymer ein Polyetherurethan oder ein Polyesterurethan ist, das antithrombogene Agens ein Heparin und der Komplexbildner Benzalkoniumchlorid ist.

13. Vorrichtung oder Teil einer Vorrichtung nach Anspruch 1, wobei das Polymer eine Verbindung mit Formel (I) umfasst 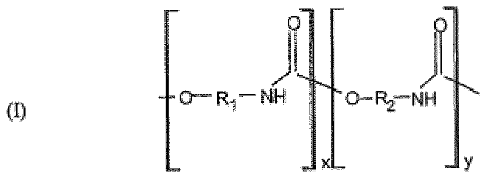 wobei R₁ und R₂ jeweils unabhängig ein gesättigter Kohlenwasserstoff, ein ungesättigter Kohlenwasserstoff, ein substituiertes Phenyl, ein nicht-substituiertes Phenyl, ein Phenoxy, ein Toluenisocyanat, ein Toluendiisocyanat, ein Polyisocyanat ist, und wobei x und y jeweils zwischen 50 und 1000 sind.

14. Vorrichtung oder Teil einer Vorrichtung nach Anspruch 13, wobei R₁ und R₂ jeweils unabhängig eine Verbindung mit Formel (II) umfassen 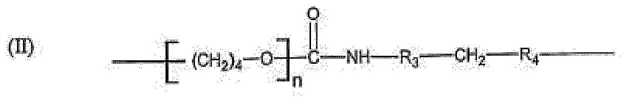 wobei R₃ und R₄ jeweils unabhängig ein aromatischer oder ein aliphatischer zyklischer Kohlenwasserstoff sind und n zwischen 1 und 500 ist.

15. Vorrichtung oder Teil einer Vorrichtung nach Anspruch 10, wobei das Polymer ein Block-Polystyren-co-Polymer ist, das antithrombogene Agens ein Heparin ist und der Komplexbildner Benzalkoniumchlorid ist.

16. Vorrichtung oder Teil einer Vorrichtung nach Anspruch 15, wobei das Block-Polystyren-co-Polymer eine Verbindung mit Formel (III) umfasst 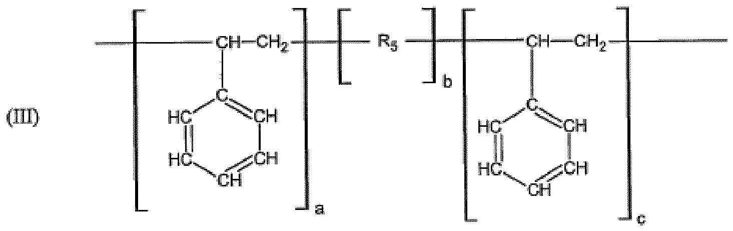 wobei R₅ ein Dien ist und wobei a, b und c jeweils unabhängig zwischen 10 und 1000 sind.

17. Vorrichtung oder Teil einer Vorrichtung nach Anspruch 13, weiter umfassend ein zusätzliches pharmakologisches Agens, das auf wenigstens eine Oberfläche des Polymers beschichtet ist.

18. Vorrichtung oder Teil einer Vorrichtung nach Anspruch 13, weiter umfassend einen Komplexbildner.

19. Vorrichtung oder Teil einer Vorrichtung nach Anspruch 1, umfassend 0,05 bis 0,5 Äquivalente des pharmakologischen Agens pro Äquivalent des Polymers.

20. Vorrichtung oder Teil einer Vorrichtung nach Anspruch 1, weiter umfassend eine Oberflächenbeschichtung auf der Zusammensetzung, wobei das pharmakologische Agens homogen in die Oberflächenbeschichtung aufgenommen worden ist.

21. Vorrichtung nach Anspruch 1, konstruiert und angeordnet zur Verwendung als ein Ventrikel eines menschlichen Herzens.

22. Vorrichtung nach Anspruch 21, wobei das Polymer ein Polyurethan und das pharmakologische Agens ein Heparin ist.

23. Vorrichtung nach Anspruch 22, wobei das Heparin mit einem Komplexbildner komplexiert ist.

24. Vorrichtung nach Anspruch 22, wobei das Heparin menschliches Heparin, Rinderheparin, Schweineheparin, Heparinsulfat, ein Heparin mit niedrigem Molekulargewicht, ein Heparinanalog, ein synthetisches Heparin, ein Heparinoid, oder eine Kombination davon ist.

25. Vorrichtung nach Anspruch 21, weiter umfassend
eine erste Kammer;
eine Einlassöffnung in Flüssigverbindung mit der ersten Kammer und ausgeführt, um Flüssigkeitsstrom in die erste Kammer zu erlauben; und
eine Auslassöffnung in Flüssigverbindung mit der ersten Kammer und ausgeführt, um Flüssigkeitsstrom von der ersten Kammer zu einem arteriellen System eines Menschen zu erlauben,
wobei entweder die Einlassöffnung oder die Auslassöffnung oder beide ein Ventil umfassen.

26. Vorrichtung nach Anspruch 21, weiter umfassend:
eine erste Kammer;
eine Einlassöffnung in Flüssigverbindung mit der ersten Kammer;
eine zweite Kammer in Flüssigverbindung mit der ersten Kammer;
ein Ventil, das ausgeführt ist, um Flüssigkeitsstrom von der ersten Kammer in die zweite Kammer zu steuern; und
eine Auslassöffnung in Flüssigverbindung mit der zweiten Kammer und
ausgeführt, um Flüssigkeitsstrom zu einem arteriellen System eines Menschen bereitzustellen.

27. Vorrichtung nach Anspruch 21, wobei alle blutkontaktierenden Oberflächen der Vorrichtung eine Zusammensetzung umfassen, die ein Polymer und ein antithrombogenes Agens umfasst, das in das Polymer imprägniert ist.

28. Ventrikelhilfsvorrichtung, konstruiert und angeordnet, um eine anhaltende Freisetzung eines pharmakologischen Agens bereitzustellen, wobei die Vorrichtungen eine Zusammenfassung umfasst, wobei die Zusammensetzung
ein Polymer; und
eine wirksame Menge eines pharmakologischen Agens umfasst, das in das Polymer imprägniert ist,
wobei das imprägnierte pharmakologische Agens in dem internen Gitternetzwerk des Polymer angeordnet oder verteilt ist, wobei die Zusammensetzung 1-5 % pharmakologisches Agens bezogen auf das Gewicht der Zusammensetzung umfasst.

29. Ventrikelhilfsvorrichtung nach Anspruch 28, weiter umfassend eine Oberflächenbeschichtung, wobei das pharmakologische Agens homogen in die Oberflächenbeschichtung eingebaut worden ist.

30. Ventrikelhilfsvorrichtung nach Anspruch 28, wobei das pharmakologische Agens ein Heparin und das Polymer ein Polyurethan ist.

31. Ventrikelhilfsvorrichtung nach Anspruch 28, weiter umfassend:
eine erste Kammer;
eine Einlassöffnung in Flüssigverbindung mit der ersten Kammer und
ausgeführt, um Flüssigkeitsstrom in die erste Kammer zu erlauben; und
eine Auslassöffnung in Flüssigverbindung mit der ersten Kammer und
ausgeführt, um Flüssigkeitsstrom von der ersten Kammer zu einem arteriellen System eines Menschen zu erlauben.

32. Ventrikelhilfsvorrichtung nach Anspruch 28, weiter umfassend:
eine erste Kammer;
eine Einlassöffnung in Flüssigverbindung mit der ersten Kammer;
eine zweite Kammer in Flüssigverbindung mit der ersten Kammer;
ein Ventil, das ausgeführt ist, um Flüssigkeitsstrom von der ersten Kammer zu der zweiten Kammer zu steuern; und
eine Auslassöffnung in Flüssigverbindung mit der zweiten Kammer und
ausgeführt, um Flüssigkeitsstrom zu einem arteriellen System eines Menschen bereitzustellen.

33. Katheter umfassend:
einen Balg umfassend eine Zusammensetzung, die ein Polymer umfasst, das mit einem ersten pharmakologischen Agens imprägniert ist, wobei das imprägnierte pharmakologische Agens in dem internen Gitternetzwerk des Polymers angeordnet oder verteilt ist, wobei die Zusammenfassung 1-5 % pharmakologisches Agens bezogen auf das Gewicht der Zusammensetzung umfasst; und
eine Röhre, die mit dem Balg verbunden ist und funktionsfähig ist, um den Balg aufzublasen.

34. Katheter nach Anspruch 33, wobei das erste pharmakologische Agens ein Heparin ist.

35. Katheter nach Anspruch 33, wobei der Balg ein Polyurethan umfasst.

36. Katheter nach Anspruch 35, wobei der Balg weiter eine Oberflächenbeschichtung umfasst, die ein zweites pharmakologisches Agens umfasst, das homogen in die Oberflächenbeschichtung aufgenommen ist, wobei das zweite pharmakologische Agens identisch mit oder verschieden sein kann von dem ersten pharmakologischen Agens, bevorzugterweise sind das erste und das zweite pharmakologische Agens jeweils ein Heparin.

37. Vorrichtung oder Teil einer Vorrichtung nach Anspruch 1, zur Verwendung bei der Behandlung von Thrombose.

38. Vorrichtung nach Anspruch 37, wobei die Vorrichtung als eine Ventrikelhilfsvorrichtung ausgeführt ist.

39. Vorrichtung nach Anspruch 37, wobei die Vorrichtung als ein Stent ausgeführt ist.

40. Vorrichtung nach Anspruch 37, wobei die Vorrichtung als ein Katheter ausgeführt ist.

## Revendications

1. Dispositif médical ou partie de dispositif médical formé à partir d'une composition comprenant :
un polymère ; et
une quantité efficace d'un agent pharmacologique imprégné dans le polymère,
dans lequel l'agent pharmacologique imprégné est disposé ou distribué dans le réseau en grille interne du polymère dans lequel la composition comprend 1 à 5 % d'agent pharmacologique en poids de la composition.

2. Dispositif ou partie d'un dispositif selon la revendication 1, dans lequel l'agent pharmacologique est un agent athrombogène.

3. Dispositif ou partie d'un dispositif selon la revendication 1, dans lequel le polymère est un polymère biocompatible.

4. Dispositif ou partie d'un dispositif selon la revendication 3, dans lequel le polymère est un polyéther, un polyuréthane, un polyester uréthane, un polyéther uréthane, un polyéther uréthane urée, un polyester, un polycarbonate, un polyéthylène de basse densité, un polyéthylène de densité moyenne, un polyéthylène de haute densité, un poly(téréphtalate d'éthylène), un poly(chlorure de vinyle), un polypropylène, un polystyrène, un polyamide, un polyacrylamide, un polyacrylate, ou une combinaison de ceux-ci.

5. Dispositif ou partie d'un dispositif selon la revendication 2, dans lequel le polymère est un polyuréthane et l'agent athrombogène est une héparine.

6. Dispositif ou partie d'un dispositif selon la revendication 5, dans lequel l'héparine est une héparine humaine, une héparine bovine, une héparine porcine, un sulfate d'héparine, une héparine de basse masse moléculaire, un analogue d'héparine, une héparine de synthèse, un héparinoïde, ou une combinaison de ceux-ci.

7. Dispositif ou partie d'un dispositif selon la revendication 5, comprenant en outre une héparine déposée sur au moins une surface du polyuréthane.

8. Dispositif ou partie d'un dispositif selon la revendication 1, comprenant en outre un agent de complexation.

9. Dispositif ou partie d'un dispositif selon la revendication 8, dans lequel l'agent de complexation est un sel de benzalkonium, un sel d'ammonium, un chlorure de tridodécylammonium, un chlorure de cétylpyrimidine, un chlorure de stérylammonium, ou une combinaison de ceux-ci.

10. Dispositif ou partie d'un dispositif selon la revendication 2, comprenant en outre un agent de complexation.

11. Dispositif ou partie d'un dispositif selon la revendication 10, dans lequel l'agent pharmacologique est une héparine humaine, une héparine bovine, une héparine porcine, un sulfate d'héparine, une héparine de basse masse moléculaire, un analogue d'héparine, une héparine de synthèse, un héparinoïde, ou une combinaison de ceux-ci.

12. Dispositif ou partie d'un dispositif selon la revendication 10 ou 11, dans lequel le polymère est un polyéther uréthane ou un polyester uréthane, l'agent athrombogène est une héparine et l'agent de complexation est le chlorure de benzalkonium.

13. Dispositif ou partie d'un dispositif selon la revendication 1, dans
lequel le polymère comprend un composé ayant la formule (I) 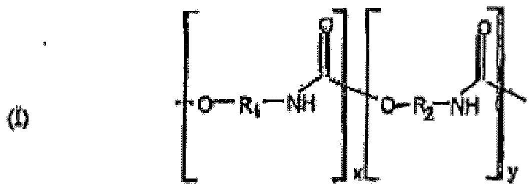 dans laquelle chacun de R₁ et R₂ représente indépendamment un hydrocarbure saturé, un hydrocarbure insaturé, un phényle substitué, un phényle non substitué, un phénoxy, un isocyanate de toluène, un diisocyanate de toluène, un poly-isocyanate, et dans laquelle x et y valent chacun entre 50 et 1000.

14. Dispositif ou partie d'un dispositif selon la revendication 13, dans lequel chacun de R₁ et R₂ comprend indépendamment un composé ayant la formule (II) 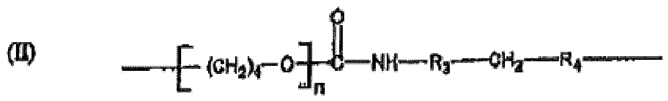 dans laquelle chacun de R₃ et R₄ représente indépendamment un hydrocarbure cyclique aromatique ou aliphatique et n vaut entre 1 et 500.

15. Dispositif ou partie d'un dispositif selon la revendication 10, dans lequel le polymère est un copolymère de polystyrène séquencé, l'agent athrombogène est une héparine et l'agent de complexation est le chlorure de benzalkonium.

16. Dispositif ou partie d'un dispositif selon la revendication 15, dans lequel le copolymère de polystyrène séquencé comprend un composé ayant la formule (III) 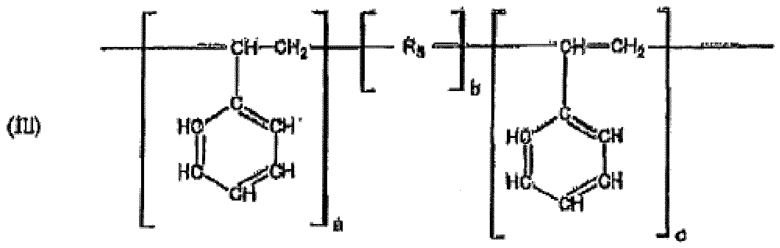 dans laquelle R₅ représente un diène, et dans laquelle chacun de a, b et c vaut indépendamment entre 10 et 1000.

17. Dispositif ou partie d'un dispositif selon la revendication 13, comprenant en outre un agent pharmacologique supplémentaire déposé sur au moins une surface du polymère.

18. Dispositif ou partie d'un dispositif selon la revendication 13, comprenant en outre un agent de complexation.

19. Dispositif ou partie d'un dispositif selon la revendication 1, comprenant 0,05 à 0,5 équivalent de l'agent pharmacologique par équivalent du polymère.

20. Dispositif ou partie d'un dispositif selon la revendication 1, comprenant en outre un enrobage de surface sur la composition dans lequel l'agent pharmacologique a été incorporé de manière homogène dans l'enrobage de surface.

21. Dispositif selon la revendication 1, construit et arrangé pour une utilisation en tant que ventricule d'un coeur humain.

22. Dispositif selon la revendication 21, dans lequel le polymère est un polyuréthane et l'agent pharmacologique est une héparine.

23. Dispositif selon la revendication 22, dans lequel l'héparine est complexée avec un agent de complexation.

24. Dispositif selon la revendication 22, dans lequel l'héparine est une héparine humaine, une héparine bovine, une héparine porcine, un sulfate d'héparine, une héparine de basse masse moléculaire, un analogue d'héparine, une héparine de synthèse, un héparinoïde, ou une combinaison de ceux-ci.

25. Dispositif selon la revendication 21, comprenant en outre :
une première chambre ;
un port d'entrée en communication liquide avec la première chambre et configuré pour permettre la circulation de liquide dans la première chambre ; et
un port de sortie en communication liquide avec la première chambre et configuré pour permettre à un liquide de circuler de la première chambre vers un système artériel d'un être humain,
dans lequel le port d'entrée et / ou le port de sortie comprend une valve.

26. Dispositif selon la revendication 21, comprenant en outre :
une première chambre ;
un port d'entrée en communication liquide avec la première chambre ;
une seconde chambre en communication liquide avec la première chambre ;
une valve configurée pour contrôler la circulation du liquide de la première chambre vers la seconde chambre ; et
un port de sortie en communication liquide avec la seconde chambre et configuré pour fournir une circulation du liquide vers un système artériel d'un être humain.

27. Dispositif selon la revendication 21, dans lequel toutes les surfaces du dispositif en contact avec le sang comprennent une composition qui comprend un polymère et un agent athrombogène imprégné dans le polymère.

28. Dispositif d'assistance ventriculaire construit et arrangé pour fournir une libération prolongée d'un agent pharmacologique, le dispositif comprenant une composition comprenant :
un polymère ; et
une quantité efficace d'un agent pharmacologique imprégné dans le polymère,
dans lequel l'agent pharmacologique imprégné est disposé ou distribué dans le réseau en grille interne du polymère, dans lequel ladite composition comprend 1 à 5 % d'agent pharmacologique en poids de la composition.

29. Dispositif d'assistance ventriculaire selon la revendication 28, comprenant en outre un enrobage de surface dans lequel l'agent pharmacologique a été incorporé de manière homogène dans l'enrobage de surface.

30. Dispositif d'assistance ventriculaire selon la revendication 28, dans lequel l'agent pharmacologique est une héparine et le polymère est un polyuréthane.

31. Dispositif d'assistance ventriculaire selon la revendication 28, comprenant en outre :
une première chambre ;
un port d'entrée en communication liquide avec la première chambre et configuré pour permettre la circulation de liquide dans la première chambre ; et
un port de sortie en communication liquide avec la première chambre et configuré pour permettre au liquide de circuler de la première chambre vers un système artériel d'un être humain.

32. Dispositif d'assistance ventriculaire selon la revendication 28, comprenant en outre :
une première chambre ;
un port d'entrée en communication liquide avec la première chambre ;
une seconde chambre en communication liquide avec la première chambre ;
une valve configurée pour contrôler la circulation de liquide de la première chambre vers la seconde chambre ; et
un port de sortie en communication liquide avec la seconde chambre et configuré pour fournir une circulation de liquide vers un système artériel d'un être humain.

33. Cathéter comprenant :
une vessie comprenant une composition qui comprend un polymère imprégné avec un premier agent pharmacologique, dans laquelle l'agent pharmacologique imprégné est disposé ou distribué dans le réseau en grille interne du polymère, dans laquelle la composition comprend 1 à 5 % d'agent pharmacologique en poids de la composition ; et
un tuyau relié à la vessie et prévu pour gonfler la vessie.

34. Cathéter selon la revendication 33, dans lequel le premier agent pharmacologique est une héparine.

35. Cathéter selon la revendication 33, dans lequel la vessie comprend un polyuréthane.

36. Cathéter selon la revendication 35, dans lequel la vessie comprend en outre un enrobage de surface comprenant un second agent pharmacologique incorporé de manière homogène dans l'enrobage de surface, dans lequel le second agent pharmacologique peut être identique ou différent du premier agent pharmacologique, de préférence les premier et second agents pharmacologiques sont chacun une héparine.

37. Dispositif ou partie d'un dispositif selon la revendication 1, pour une utilisation dans le traitement de la thrombose.

38. Dispositif selon la revendication 37, où le dispositif est configuré sous la forme d'un dispositif d'assistance ventriculaire.

39. Dispositif selon la revendication 37, où le dispositif est configuré sous la forme d'un stent.

40. Dispositif selon la revendication 37, où le dispositif est configuré sous la forme d'un cathéter.
